(19) Europäisches Patentamt
European Patent Office
Office européen des brevets

(11) **EP 1 927 317 A1**

(12) **EUROPEAN PATENT APPLICATION**
published in accordance with Art. 153(4) EPC

(43) Date of publication:
**04.06.2008 Bulletin 2008/23**

(51) Int Cl.:
***A61B 8/08*** *(2006.01)*

(21) Application number: **06798005.2**

(22) Date of filing: **14.09.2006**

(86) International application number:
**PCT/JP2006/318293**

(87) International publication number:
**WO 2007/034738 (29.03.2007 Gazette 2007/13)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **20.09.2005 JP 2005272571**

(71) Applicant: **MATSUSHITA ELECTRIC INDUSTRIAL CO., LTD.**
**Osaka 571-8501 (JP)**

(72) Inventors:
• **SUZUKI, Takao**
c/o Matsuhita Electric Ind. Co., Ltd.
**2-1-61, Shiromi, Chuo-ku, Osaka 540-6207 (JP)**
• **KATO, Makoto**
c/o Matsuhita Electric Ind. Co., Ltd.
**2-1-61, Shiromi, Chuo-ku, Osaka 540-6207 (JP)**
• **HAGIWARA, Hisashi**
c/o Matsuhita Electric Ind. Co., Ltd.
**2-1-61, Shiromi, Chuo-ku, Osaka 540-6207 (JP)**
• **TAN-NAKA, Yoshinao**
c/o Matsuhita Electric Ind. Co., Ltd.
**2-1-61, Shiromi, Chuo-ku, Osaka 540-6207 (JP)**

(74) Representative: **Grünecker, Kinkeldey, Stockmair & Schwanhäusser Anwaltssozietät Leopoldstrasse 4 80802 München (DE)**

(54) **ULTRASONIC DIAGNOSTIC EQUIPMENT**

(57) The present invention aims at providing an ultrasonic diagnostic apparatus capable of acquiring more accurate tissue character values such as strain, an average elastic modulus, and an average viscosity.

The ultrasonic diagnostic apparatus of the present invention detects tissue boundaries of a subject in accordance with a received signal based on an ultrasonic echo from a receiving section 103, by means of a boundary detection section 110; and tracks motions of the tissue boundaries of the subject by means of a tissue tracking section 108. A tissue character value computing section 109 computes tissue character values of the subject from the motions of the tissue boundaries of the subject tracked by the tissue tracking section 108 and a blood pressure value of the subject acquired from a blood pressure value acquisition section 112.

*FIG. 1*

Printed by Jouve, 75001 PARIS (FR)

**Description**

Technical Field

The present invention relates to an ultrasonic diagnostic apparatus which measures a tissue character of a subject.

Background Art

A related-art ultrasonic diagnostic apparatus emits an ultrasonic wave to a subject and converts the intensity of a reflected echo signal into the brightness of a corresponding pixel, thereby acquiring the structure of the subject in the form of a tomographic image. An attempt has recently been made to precisely measure motion of a subject by means of analyzing primarily the phase of a reflected echo signal, thereby determining tissue characters such as the strain, an elastic modulus, and viscosity of a tissue of the subject.

Patent Document 1 describes a method for tracking the tissue of the subject with high accuracy by determining the instantaneous position of the subject through use of both the amplitude and phase of a detection wave output signal of a reflected echo signal, to thus capture minute vibrations in large amplitude displacement movement induced by pulsation.

The method for tracking a subject tissue described in Patent Document 1 is described by reference to Fig. 6. Received echo signals of ultrasonic pulses transmitted at an interval $\Delta T$ in a single direction with regard to the subject are taken as $Y(t)$ and $y(t+AT)$. Provided that a pulse transmission time is $t = 0$ and sound speed is taken as $C$, a time "$t_x$" at which an echo reflected from a point of measurement set at a certain position (depth) "$x$" is received is defined as $t_x = x/(C/2)$. At that time, provided that a phase difference between $y(t_x)$ and $y(t_x+AT)$ is taken as $\Delta\theta$ and that the center frequency of ultrasonic waves achieved in the vicinity of $t_x$ is taken as "$f$," the amount of movement $Ax$ of the point of measurement achieved during the period $AT$ is expressed by (Eq. 1).

$$\Delta x = -C \bullet \Delta\theta / 4\pi f \quad \dots \text{(Eq. 1)}$$

By addition of the amount of movement to "$x$," the position (depth) $x'$ of the point of measurement after elapse of $\Delta T$ can be determined by (Eq. 2).

$$x' = x + \Delta x \quad \dots \text{(Eq. 2)}$$

The position of the point of measurement in the subject can be tracked by repetition of the above operations. In short, provided that a, time at which an echo reflected from the depth $x'$ is received is taken as $t'$ and that a subsequently-transmitted-and-received signal is taken as $y(t+2\Delta T)$, a position $x''$ of the point of measurement achieved after elapse of $2\Delta T$ can be determined from a phase difference $\Delta\theta'$ between $y(t_x'+\Delta T)$ and $y(t_x'+2\Delta T)$ through arithmetic operations of (Eq. 1) and (Eq. 2).

Patent Document 2 describes a method that is a further development of the method described in connection with Patent Document 1 and that precisely tracks large amplitude displacement movements of a point of measurement set on a vascular wall induced by a heartbeat, measures the amount of strain in the vascular wall from a resultant difference, and determines a local elastic modulus from the amount of strain and a blood pressure difference, as well as describing an apparatus for displaying a spatial distribution of elastic modulus as an image.

The elastic modulus computing method described in Patent Document 2 is described by reference to Fig. 7. According to Patent Document 2, a probe 101 emits ultrasonic waves to a subject and receives an echo from a blood vessel, particularly, an echo from an artery. Points of measurement A, B, and C are set on the blood vessel in a depthwise direction while being spaced apart from each other at a uniform interval Ws; signals received from the points of measurement A, B, and C are analyzed by means of the method described in Patent Document 1; and movements of the points of measurement A, B, and C are tracked. The artery repeats contraction and expansion by means of a heartbeat, and therefore movements of the points of measurement A, B, and C also become periodic as described by tracking waveforms TA, TB, and TC. The movements include rapid expansion of a vascular wall in a heart contraction phase and slow contraction of the blood vessel in a heart expansion phase.

A thickness change waveform WAB appearing in an area between points of measurement A and B and a thickness change waveform WBC appearing in an area between points of measurement B and C are determined from the tracking waveforms TA, TB, and TC. Provided that the amount of a change in the thickness change waveform WAB is ΔW(AB), the amount of strain ε(AB) appearing in the area between the points of measurement A and B is expressed by (Eq. 3).

$$\varepsilon(AB) = \Delta W(AB)/Ws \qquad \dots (Eq.\ 3)$$

On the assumption that there stands a relationship of a blood pressure difference ΔP achieved at this time = (the highest blood pressure) - (the lowest blood pressure), an elastic modulus E(AB) achieved between the points of measurement A and B can be determined by (Eq. 4).

$$E(AB) = \Delta P/\varepsilon(AB) = \Delta P \cdot Ws/\Delta W(AB) \qquad \dots (Eq.\ 4)$$

Likewise, an elastic modulus E(BC) acquired between the points of measurement B and C can be determined. Further, the same operation is performed in connection with a plurality of points on a tomographic image, whereby an image pertaining to the distribution of elastic moduli is obtained.

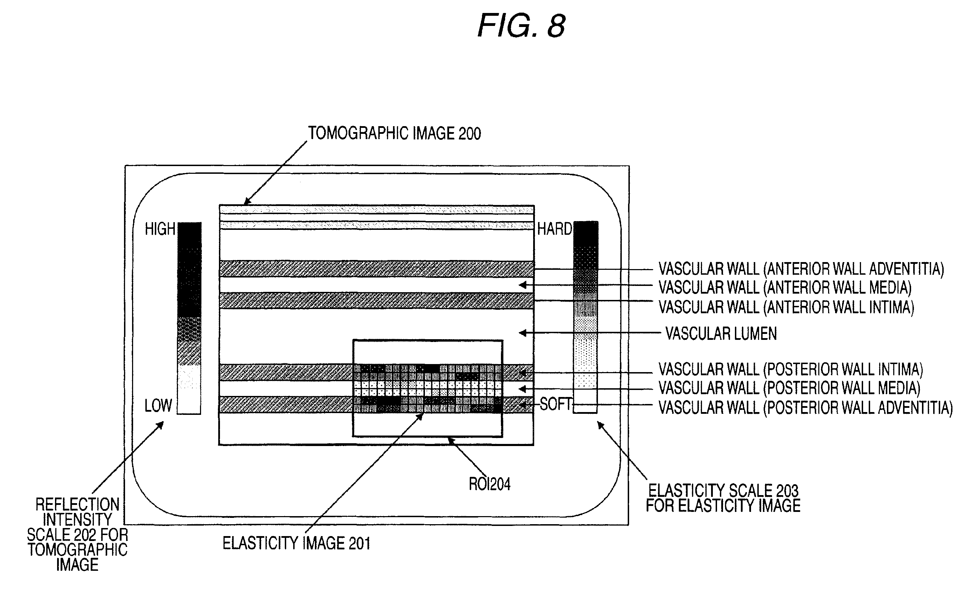

Fig. 8 is an example of an actual diagnostic screen achieved in the related art. A screen shows a longitudinally-split cross section of a blood vessel. An area (ROI) 204 for computing an elastic modulus is set on a monochrome tomographic image 200 appearing on the screen, and a plurality of points of measurement are set at an uniform interval within the ROI 204 in the vertical direction and at another uniform interval within the ROI 204 in the horizontal direction. Movements of all of the points of measurement are computed by (Eq. 1) and (Eq. 2), and an elastic modulus achieved between points of measurement; namely, an elastic modulus of a minute area in the vascular wall, is computed by (Eq. 3) and (Eq. 4). The thus-acquired elastic moduli are converted into color codes and located at predetermined positions, thereby creating an elastic modulus image 201. The elastic modulus image 201 is displayed, in a superimposed manner, on a corresponding position on the monochrome tomographic image 200. As a result, the distribution of elastic moduli acquired in the vascular wall can be observed in detail. A vertical distance between the points of measurement is of the order of tens of micrometers, and a horizontal distance between the points of measurement is of the order of several tens and a hundred of micrometers. Moreover, a reflection intensity scale 202 for a tomographic image showing a relationship between an amplitude of a received signal and the brightness on a screen and an elastic modulus scale 203 for an elastic modulus image showing a relationship between an elastic modulus and a color on a screen are displayed on the screen. Further, although unillustrated, various types of information useful for diagnosis, such as an average elastic modulus value, a diameter of a blood vessels, and an electrocardiographic waveform, are also displayed on the screen.

Correspondence between an elastic modulus and an actual disease is still in the studying stage, but Non-Patent Document 1 discloses, by way of example, relevance between an average elastic modulus and a risk factor of an arteriosclerotic disease.

Patent Document 1: JP-A-10-5226
Patent Document 2: JP-A-2000-229078
Non-Patent Document 1: Okimoto *et al.,* "Relevance between an elastic characteristic of a carotid artery and a risk factor of an arteriosclerotic disease determined by a new diagnostic method" the 42nd Regular Meeting held at Tohoku Subdivision of Japan Diabetes Society.

Disclosure of the Invention

Problem that the Invention is to solve

However, the average elastic modulus disclosed in Non-Patent 1 is acquired by fixing a standard thickness Ws and averaging elastic moduli of minute areas, each of which has a fixed size, over a required region. In the meantime, an average elastic modulus of the vascular wall shown in Fig. 8 is determined by averaging elastic moduli of minute areas on the vascular wall. In general, in relation to an average elastic modulus of a target area, a modulus determined from strain in and force exerted on the entire target area is considered to be a correct average modulus. When an area is divided into subdivisions and when an average elastic modulus of the entirety is determined by averaging elastic moduli determined from strain in and force exerted on the subdivisions, the average elastic modulus contains an error when compared with a correct average elastic modulus.

The present invention has been conceived in light of the circumstance and aims at providing an ultrasonic diagnostic apparatus capable of providing more accurate tissue character values, such as strain, an average elastic modulus, and an average viscosity:

Means for Solving the Problem

An ultrasonic diagnostic apparatus of the present invention has a receiving section that outputs a received signal based on an ultrasonic echo reflected from inside of a subject exposed to ultrasonic waves; a tissue tracking section that tracks motions of tissue boundaries of the subject in accordance with the received signal; and a tissue character value computing section that computes tissue character values of the subject from the motions of the tissue boundaries of the subject tracked by the tissue tracking section. According to the present invention, a more accurate average tissue character value determined from strain in an overall area can be obtained.

An ultrasonic diagnostic apparatus of the present invention has a receiving section that outputs a received signal based on an ultrasonic echo reflected from inside of a subject exposed to ultrasonic waves; a boundary detection section that detects tissue boundaries of the subject from the received signal; a tissue tracking section that tracks, in accordance with the received signal, motions of the tissue boundaries of the subject detected by the boundary detection section; and a tissue character value computing section that computes tissue character values of the subject from the motions of the tissue boundaries of the subject tracked by the tissue tracking section. According to the present invention, a more accurate average tissue character value determined from strain in an overall area can be obtained.

An ultrasonic diagnostic apparatus of the present invention has a receiving section that outputs a received signal based on an ultrasonic echo reflected from inside of a subject exposed to ultrasonic waves; a tissue tracking section that tracks motions of tissue of the subject from the received signal; a tissue boundary detection section that detects tissue boundaries of the subject in accordance with at least either the received signal or the motions of the tissues of the subject tracked by the tissue tracking section; and a tissue character value computing section that computes tissue character values of the subject from the motions of the tissue of the subject corresponding to the tissue boundaries of the subject detected by the tissue boundary detection section. According to the present invention, a more accurate average tissue character value determined from strain in an overall area can be obtained.

The ultrasonic diagnostic apparatus of the present invention has at least two tissue character value computing modes, wherein tissue character values determined from motions of the tissue boundaries of the subject are output in a first tissue character value computing mode; and tissue character values of a minute area of a fixed size of the subject are determined and output in another tissue character value computing mode. According to the present invention, average tissue character values of two different types can be determined.

The ultrasonic diagnostic apparatus of the present invention further has a memory that stores the received signal, wherein, when a computing mode is switched in a freeze state, tissue character values obtained in a computing mode achieved after switching are output in accordance with a received signal read from the memory. According to the present invention, a plurality of types of tissue character values can be obtained without involvement of re-measurement.

The ultrasonic diagnostic apparatus of the present invention further has a display section for displaying tissue character values of the subject, wherein the display section simultaneously displays tissue character values determined from the motions of the tissue boundaries of the subject and the tissue character values computed in connection with the minute area of the fixed size in the subject. According to the present invention, average tissue character values of two different types can be ascertained simultaneously.

In the ultrasonic diagnostic apparatus of the present invention, the tissue character values determined from the motions of the tissue boundaries are numerals, and the tissue character values computed in connection with the minute area of the fixed size are displayed in the form of a distribution image. According to the present invention, average tissue character values of two different types can be ascertained simultaneously.

The ultrasonic diagnostic apparatus of the present invention further has a blood pressure value acquisition section that acquires a blood pressure value of the subject, wherein the tissue character value computing section computes at least either an elastic modulus or a viscosity as a tissue characteristic value of the subject from the motions of the tissue boundaries of the subject and the blood pressure value acquired by the blood pressure value acquisition section. According to the present invention, there can be obtained tissue character values which make it easy to ascertain characters of a subject tissue determined from strain in an overall area and pressure exerted on the area.

Advantage of the Invention

The present invention can provide more accurate tissue character values such as strain, an average elastic modulus, and an average viscosity.

Brief Description of the Drawings

Fig. 1 is a block diagram of an ultrasonic diagnostic apparatus of a first embodiment of the present invention.
Fig. 2 is a view for describing operation of the ultrasonic diagnostic apparatus of the first embodiment of the present invention.
Fig. 3 is a view for describing operation of the ultrasonic diagnostic apparatus of the first embodiment of the present invention.
Fig. 4 is a block diagram of an ultrasonic diagnostic apparatus of a second embodiment of the present invention.
Fig. 5 is a view for describing operation of the ultrasonic diagnostic apparatus of the second embodiment of the present invention.

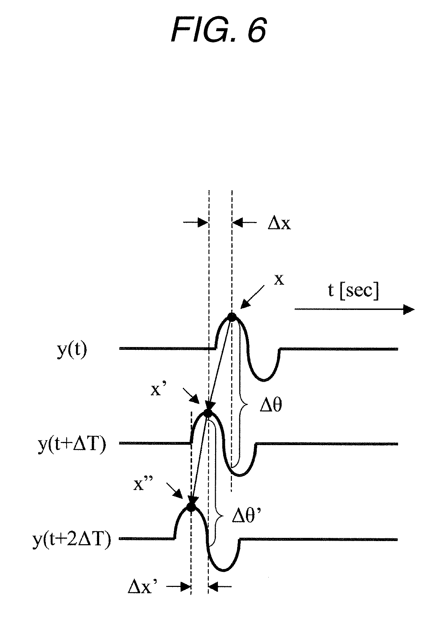

Fig. 6 is a view for describing a method for tracking a subject tissue by utilization of a signal output as a result of detection of a reflected echo signal.

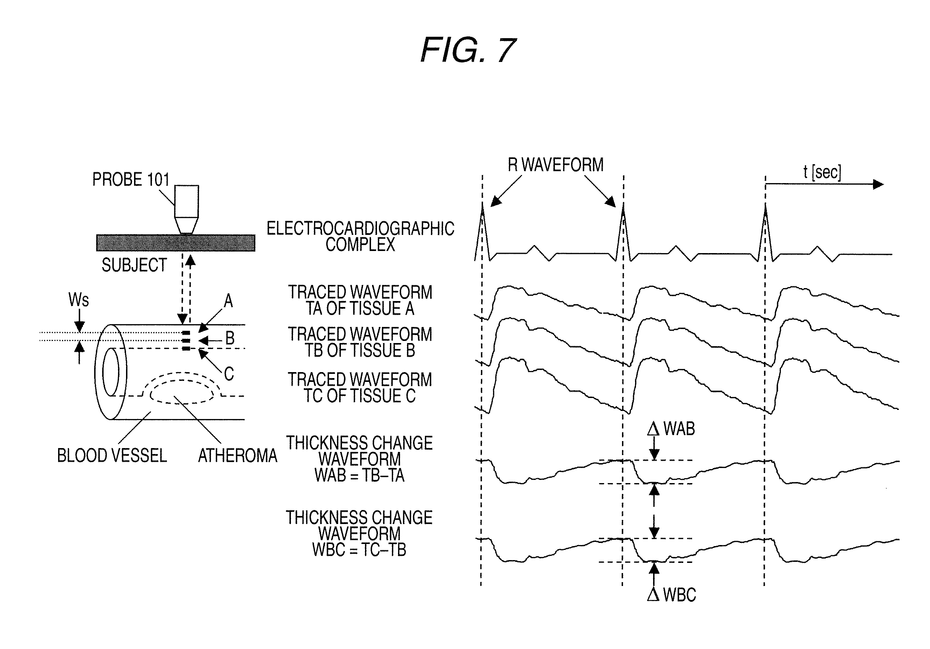

Fig. 7 is a view for describing a method for determining the amount of strain from a tissue track waveform.
Fig. 8 is a view showing an example of a monitor screen of a related-art ultrasonic diagnostic apparatus.

Descriptions of the Reference Numerals

100    CONTROL SECTION
101    PROBE
102    TRANSMISSION SECTION
103    RECEIVING SECTION
104    TOMOGRAPHIC IMAGE PROCESSING SECTION
105    MEMORY
106    IMAGE SYNTHESIS SECTION
107    MONITOR
108    TISSUE TRACKING SECTION
109    TISSUE CHARACTER VALUE COMPUTING SECTION
110    BOUNDARY DETECTION SECTION
111    MEMORY
112    BLOOD PRESSURE VALUE ACQUISITION SECTION
113    TISSUE TRACKING SECTION
114    TISSUE CHARACTER VALUE COMPUTING SECTION
115    BOUNDARY DETECTION SECTION
200    TOMOGRAPHIC IMAGE
201    ELASTIC MODULUS IMAGE
202    REFLECTION INTENSITY SCALE FOR TOMOGRAPHIC IMAGE
203    ELASTIC MODULUS SCALE FOR ELASTIC MODULUS IMAGE
204    ROI
205    BLOOD FLOW - INTIMA
206    MEDIA-ADVENTITIA BOUNDARY
207    POINT OF MEASUREMENT

Best Modes for Implementing the Invention

Embodiments of the present invention will be described hereunder by use of the drawings.

(First Embodiment)

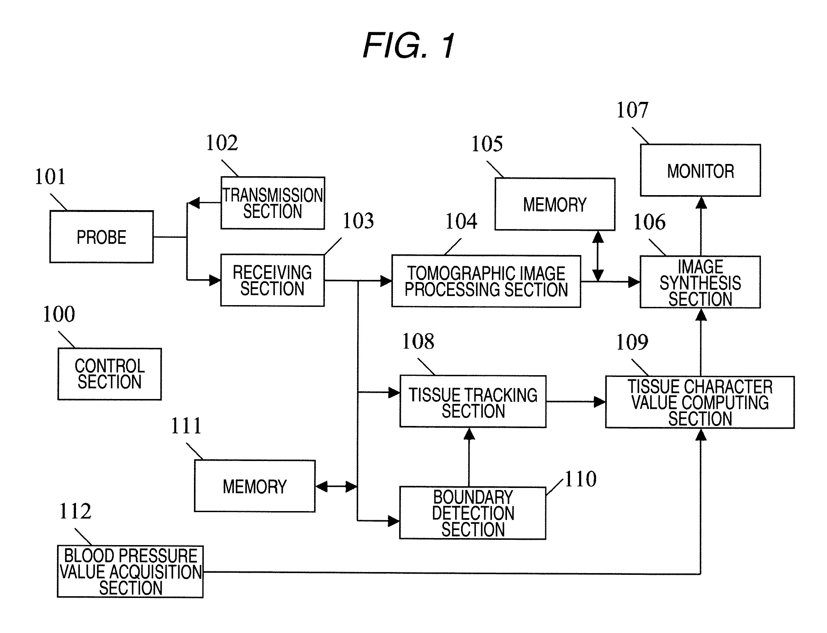

Fig. 1 is a block diagram of an ultrasonic diagnostic apparatus of a first embodiment of the present invention.

An ultrasonic diagnostic apparatus shown in Fig. 1 includes a control section 100; a probe 101; a transmission section 102; a receiving section 103; a tomographic image processing section 104; memory 105; an image synthesis section 106; a monitor 107; a tissue tracking section 108; a tissue character value computing section 109; a boundary detection section 110; memory 111; and a blood pressure value acquisition section 112.

The control section 100 controls the entire ultrasonic diagnostic apparatus. Although unillustrated, a user interface such as a keyboard, a trackball, switches, and buttons, is also connected to the control section 100.

Upon receipt of a command from the controls section 100; the transmission section 102 generates a high-voltage transmission signal for driving the probe 101 at designated timing. The probe 101 converts the transmission signal generated by the transmission section 102 into ultrasonic waves and emits the ultrasonic waves to a subject, and also converts an ultrasonic echo reflected from the inside of the subject into an electrical signal. A plurality of piezoelectric transducers are provided in the probe 101, and a deflection angle and focusing of ultrasonic waves to be transmitted are controlled by selection of the piezoelectric transducers and timing at which a voltage is applied to the piezoelectric transducers.

The receiving section 103 amplifies a received signal obtained as a result of conversion of the ultrasonic echo into an electrical signal, applies different delays to signals received by the respective piezoelectric transducers, and adds up the signals, whereupon a received signal originating from only ultrasonic waves from a given position (focus) or direction (deflection angle) is output.

The tomographic image processing section 104 is made up of a filter, a detector, a logarithmic amplifier, and the like, and analyzes at least an amplitude of a received signal output from the receiving section 103, thereby visualizing the internal structure of the subject. The boundary detection section 110 analyzes at least the amplitude of the received signal, thereby detecting a boundary of the tissue of the subject, specifically, at least any of a blood flow-intima boundary, a media-adventitia boundary, and an adventitia-peripheral tissue boundary in the vascular wall, and outputting the position of the boundary to the tissue tracking section 108. The tissue tracking section 108 analyzes at least the phase of the received signal, thereby tracking motions of points of measurement. For example, motions of the points of measurement may also be tracked by use of (Eq. 1) and (Eq. 2) provided in the related-art example. A point of measurement is a boundary position output from the boundary detection section 110 or positions set at regular intervals in a depthwise direction.

The blood pressure value acquisition section 112 is means for acquiring a blood pressure value, and may also be a keyboard used by the subject to manually input a blood pressure value or a connected blood pressure gauge.

The tissue character value computing section 109 computes the amount of strain expressed by (Eq. 3) from the tracked motions of the boundaries of the subject tissue, and computes either an elastic modulus E expressed by (Eq. 4) or a viscosity $\eta$ expressed by (Eq. 5) from the thus-acquired amount of strain and the blood pressure value.

$$P = \eta d\varepsilon/dt + E\varepsilon \; \ldots \; (Eq. \; 5)$$

The thus-acquired elastic modulus E or the viscosity $\eta$ is output as a numeral showing a tissue character or a tissue character distribution image.

The image synthesis section 106 superimposes a tomographic image on at least a numeral showing a tissue character or a tissue character distribution image, and displays a resultant image on the monitor 107.

The memory 111 stores a received signal and is utilized for re-computing a tissue character value during stoppage of transmission/receipt of ultrasonic waves (hereinafter called a "freeze state"). The memory 105 is for storing a tomographic image and outputs a tomographic image synchronizing to the tissue character value in a freeze state.

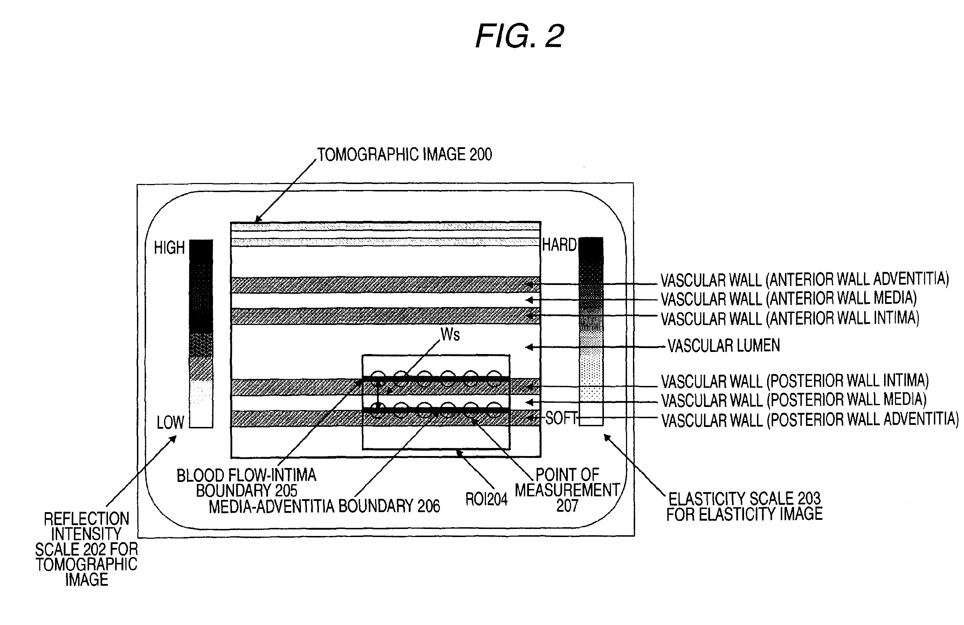

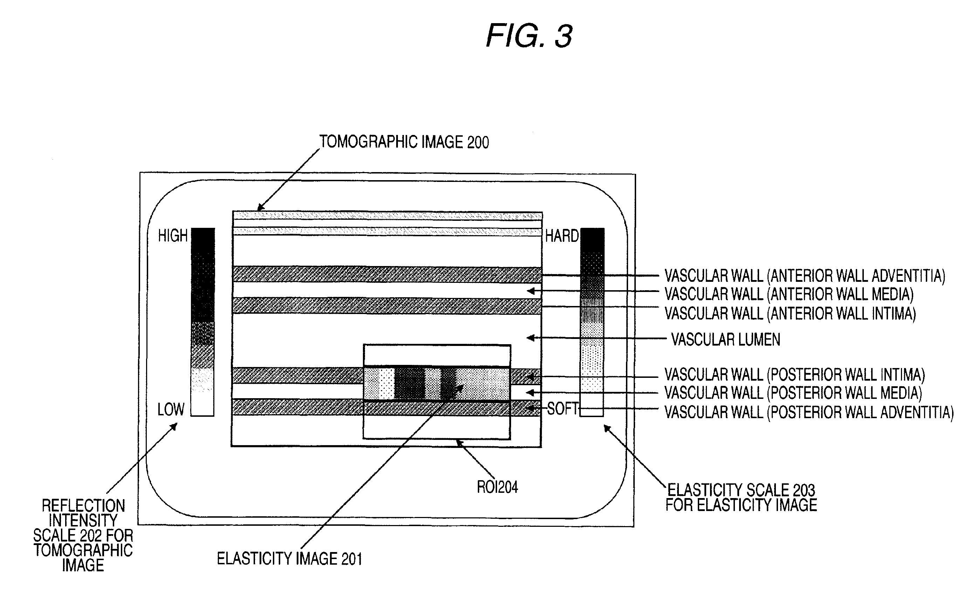

Operation of the ultrasonic diagnostic apparatus configured as mentioned above will be described by reference to Figs. 2 and 3. In Figs. 2 and 3, an elastic modulus is utilized as a tissue character value. However, the tissue character value is not limited to the elastic modulus, and another tissue character value, such as strain or a viscosity, may also be utilized. When strain is utilized, a blood pressure value is not required, and hence the blood pressure value acquisition section 112 can be omitted.

Figs. 2 and 3 are diagnostic screens showing a longitudinary-split cross section of a blood vessel analogous to Fig. 8. A region (ROI) 204 for which a tissue character value is computed is set on a monochrome tomographic image 200 to be displayed on the screen.

In this ultrasonic diagnostic apparatus, the boundary detection section 110 first analyzes a received signal, thereby detecting the blood flow-intima boundary 205 and the media-adventitia boundary 206. A boundary may also be detected by analysis of a received signal or may be manually input by the subject through use of a trackball. By way of example, since a received signal exhibits a small amplitude in a blood flow or a media, a point where a sharp increase in an amplitude is found as a result of analysis of an amplitude of the received signal is taken as a boundary. Alternatively, a blood flow may also be detected by use of a color Doppler technique, or the like, thereby detecting the blood flow-intima boundary 205. Incidentally, when the position of a boundary is clear enough to dispense with detection, the boundary detection section 115 is not provided, and information may be registered in advance in the apparatus, or the subject may input information before measurement. For instance, when the position is specified by means of modality other than ultrasonic waves, information may also be acquired from the modality. When the position of the boundary lies in the surface of a skin and when a standoff material is used between the probe 101 and the skin, the size of the standoff material may also be set previously.

The tissue tracking section 108 sets points of measurement on the boundary detected by the boundary detection section 110 or in the vicinity of the boundary, and tracks motions of the points of measurement. Fig. 2 shows points of measurement by means of outlined circles on the boundary. The tissue character value computing section 109 computes the amount of strain expressed by (Eq. 3) from the tracked motions of the boundary of the subject tissue. However, at this time, the reference thickness Ws is assumed to correspond to a width of an area between the blood flow-intima boundary 205 and the media-adventitia boundary 206 (a width indicated by a double-headed arrow in Fig. 2). As a result, an accurate average tissue character value of the area between the boundaries (the area of the intima and the area of the media in the blood vessel of Fig. 2) can be determined.

Fig. 3 is a diagnostic screen on which tissue character values converted into color codes are superimposed on the tomographic image 200. In the embodiment shown in Fig. 3, spaces between the boundaries are displayed in the same color with respect to the vertical direction. The tissue character values are averaged in a horizontal direction; whereby a more-accurate average tissue character value of the vascular wall can be determined. Since a received signal usually exhibits a large amplitude at a boundary, a large signal-to-noise ratio is attained, and motion of the tissue can be tracked more accurately when compared with another area. Therefore, a more accurate tissue character value can be determined.

The tissue tracking section 108 sets a plurality of points of measurement at regular intervals in the ROI 204 rather than on the boundary detected by the boundary detection section I 10, whereby tissue character values of respective minute areas of the vascular wall can be determined as illustrated in the related-art example. The distribution of tissue character values in the vascular wall can be observed in detail.

(Second Embodiment)

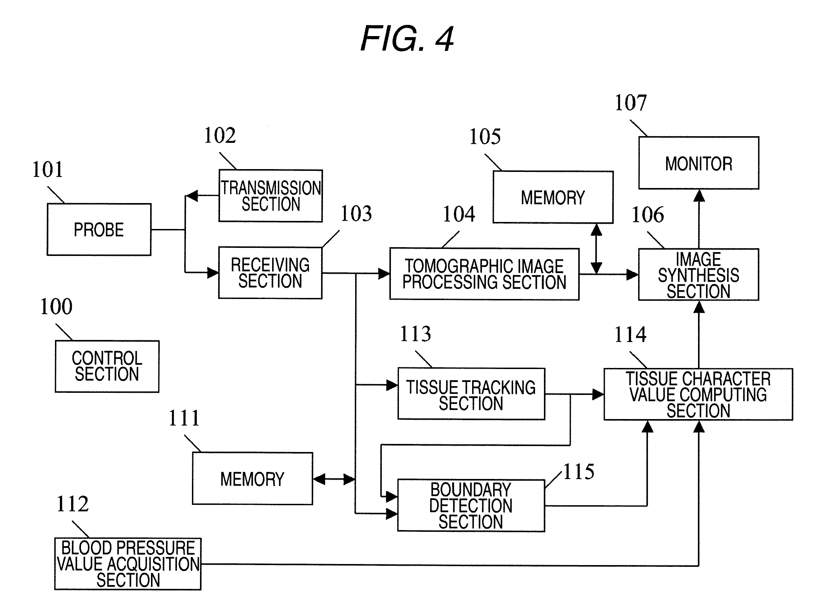

Fig. 4 is a block diagram of an ultrasonic diagnostic apparatus of a second embodiment of the present invention. Elements which are common to the ultrasonic diagnostic apparatus shown in Fig. 1 are assigned the same reference numerals, and their explanations are omitted.

The tissue tracking section 113 tracks motions of the points of measurement set at regular intervals in the depthwise direction on the basis of a phase difference between received signals, through use of (Eq. 1) and (Eq. 2). The boundary detection section 115 analyzes at least either the received signal or tracked motions of the points of measurement, to thus detect boundaries of a tissue, specifically, at least any of the blood flow-intima boundary, the media-adventitia boundary, and the adventitia-peripheral tissue boundary of the vascular wall; and specifies which one of the points of measurement tracked by the tissue tracking section 113 corresponds to the position of the boundary. The tissue character value computing section 114 computes the amount of strain expressed by (Eq. 3) from the motions of the points of measurement specified by the boundary detection section 113, and computes at least either the elastic modulus expressed by (Eq. 4) or the viscosity expressed by (Eq. 5) from the acquired amount of strain and the blood pressure value. The thus-acquired elastic modulus or viscosity is output as a numeral showing a tissue character or a tissue character distribution image.

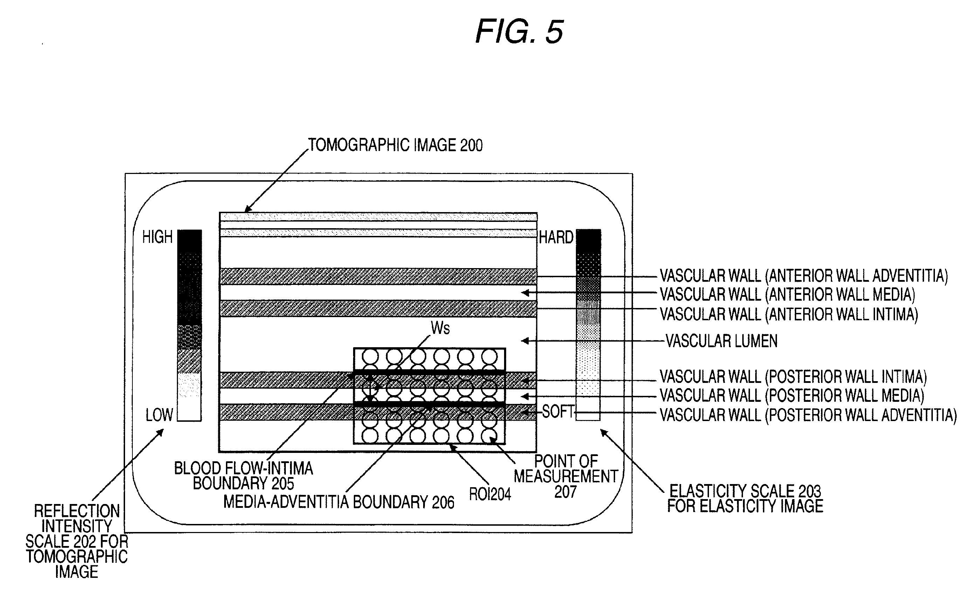

Operation of the ultrasonic diagnostic apparatus configured as mentioned above will be described by use of Fig. 5. In Fig. 5, the elastic modulus is utilized as a tissue character value, but the tissue character value is not limited to the elastic modulus. Another tissue character value such as strain or a viscosity may also be utilized. When strain is utilized, the blood pressure value is obviated, and hence the blood pressure value acquisition section 112 can be omitted.

Fig. 5 is a diagnostic screens showing a longitudinary-split cross section of a blood vessel analogous to Fig. 8. The region (ROI) 204 for which a tissue character value is computed is set on the monochrome tomographic image 200 to be displayed on the screen, and a plurality of points of measurement are set at regular intervals within the ROI 204 in both the vertical and horizontal directions. In Fig. 5, the points of measurement are designated by outlined circles.

The tissue tracking section 113 tracks motions of all of the points of measurement by use of (Eq. 1) and (Eq. 2) as in the related-art example. The boundary detection section 115 analyzes at least the received signal or the tracked motion of the subject tissue, to thus specify the blood flow-intima boundary 205 and the media-adventitia boundary 206, and specifies which one of the points of measurement tracked by the tissue tracking section 113 corresponds to the position of the boundary. The boundary may also be detected by analysis of a received signal or may be manually input by the subject through use of a trackball. By way of example, in addition to the method using the amplitude of a received signal, there may also be utilized the fact that motion of a blood-flow area involves much noise and that the motion of a vascular-wall area involves small noise. Moreover, there may also be utilized the fact that, in the heart contraction phase, the blood-flow area moves toward the probe under influence of multiple echoes reflected from a vascular anterior wall, and that an intima of a vascular posterior wall moves in an opposite direction. Further, although unillustrated, there may also be utilized the fact that elastic moduli among between the points of measurement set at regular intervals determined by the tissue character value computing section 114 are low at the blood-flow area and high at the intima of the blood vessel. In Fig. 5, the second point of measurement from the top and the third point of measurement from the bottom are specified as points of measurement on the boundaries.

The tissue character value computing section 114 computes the amount of strain expressed by (Eq. 3) from the motions of the points of measurement specified by the boundary detection section 110. At this time, the reference thickness Ws is assumed to correspond to a distance between specified points of measurement; namely, a width of an area between the blood flow-intima boundary 205 and the media-adventitia boundary 206 (a width indicated by a double-headed arrow in Fig. 5). As a result, an accurate average tissue character value of the area between the boundaries (the area of the intima and the area of the media in the blood vessel of Fig. 5)can be determined. Since a received signal usually exhibits a large amplitude at a boundary, a tissue of the boundary can be tracked more accurately when compared with the case of a tissue in another area. Therefore, a more accurate tissue character value can be determined.

The tissue character value computing section 114 determines tissue character values achieved among all of points of measurement in the ROI 204. As a result, tissue character values in respective minute areas of the vascular wall can be determined as expressed by the related-art example, so that the distribution of tissue character values in the vascular wall can be observed in detail.

Moreover, at least two tissue character value computing modes may be provided, whereby the control section 100 may also control the modes. For instance, an average tissue character value mode and a tissue character value distribution mode are provided as the tissue character computing modes. As described in connection with the foregoing embodiments, the average tissue character value mode is a mode for determining an average tissue character value from strain and a blood pressure of an entire area on the assumption that an area enclosed by boundaries of the vascular wall is taken as one area. The tissue character value distribution mode is a mode for determining tissue character values of minute areas of fixed size in the vascular wall as illustrated in the related-art example (see Non-Patent Document 1).

The subject may switch control of the modes performed by the control section 100 through use of a user interface, or a two-screen display may also be embodied by simultaneous computation of the modes. As mentioned above, the average tissue character value mode and the tissue character value distribution mode are provided, and control is performed such that the distribution of tissue character values can be observed in detail. As a result, a useful diagnosis can be carried out.

When displayed on the monitor 107, a numeral computed by the above-mentioned method may also be displayed as the average tissue character value, and the distribution of tissue character values determined by the method described in connection with the related art may also be displayed in color on a tomographic image in a superimposed manner.

Descriptions about Figs. 2, 3, and 5 provide explanations about the average tissue character value of the intima and the media in the vascular wall. However, an average tissue character value of the overall vascular wall may also be determined by use of the blood flow-intima boundary and the adventitia-peripheral tissue boundary, or an average tissue character value of the adventitia may also be determined by use of the media-adventitia boundary and the adventitia-peripheral tissue boundary.

By means of switching operation performed in a freeze state, a tomographic image stored in the memory 105 and the received signal stored in the memory 111 may also be read, re-computed, and re-displayed. As a result, two types of tissue character values can be acquired without performance of re-measurement.

Although the present invention has been described in detail or by reference to the specific embodiments, it is manifest to those skilled in the art that the present invention is susceptible to various alterations or modifications without departing from the spirit and scope of the present invention.

The present invention is based on Japanese Patent Application (JP-A-2005-272571) filed on September 20, 2005, the contents of which are hereby incorporated by reference.

Industrial Applicability

As mentioned above, according to the present invention, more accurate tissue character values such as strain, an average elastic modulus, or an average viscosity, can be obtained, and the invention is useful as an ultrasonic diagnostic apparatus for measuring a tissue character of a subject tissue.

**Claims**

1. An ultrasonic diagnostic apparatus comprising:

a receiving section that outputs a received signal based on an ultrasonic echo reflected from inside of a subject exposed to ultrasonic waves;

a tissue tracking section that tracks motions of tissue boundaries of the subject in accordance with the received signal; and

a tissue character value computing section that computes tissue character values of the subject from the motions of the tissue boundaries of the subject tracked by the tissue tracking section.

2. An ultrasonic diagnostic apparatus comprising:

a receiving section that outputs a received signal based on an ultrasonic echo reflected from inside of a subject exposed to ultrasonic waves;

a boundary detection section that detects tissue boundaries of the subject from the received signal;

a tissue tracking section that tracks, in accordance with the received signal, motions of the tissue boundaries of the subject detected by the boundary detection section; and

a tissue character value computing section that computes tissue character values of the subject from the motions of the tissue boundaries of the subject tracked by the tissue tracking section.

3. An ultrasonic diagnostic apparatus comprising:

a receiving section that outputs a received signal based on an ultrasonic echo reflected from inside of a subject exposed to ultrasonic waves;

a tissue tracking section that tracks motions of tissue of the subject from the received signal;

a tissue boundary detection section that detects tissue boundaries of the subject in accordance with at least either the received signal or the motions of the tissues of the subject tracked by the tissue tracking section; and

a tissue character value computing section that computes tissue character values of the subject from the motions of the tissue of the subject corresponding to the tissue boundaries of the subject detected by the tissue boundary detection section.

4. The ultrasonic diagnostic apparatus according to any one of claims 1 through 3, further comprising:

at least two tissue character value computing modes, wherein tissue character values determined from motions of the tissue boundaries of the subject are output in a first tissue character value computing mode; and

tissue character values of a minute area of a fixed size of the subject are determined and output in another tissue character value computing mode.

5. The ultrasonic diagnostic apparatus according to claim 4, further comprising:

memory that stores the received signal, wherein, when a computing mode is switched in a freeze state, tissue character values obtained in a computing mode achieved after switching are output in accordance with a received signal read from the memory.

6. The ultrasonic diagnostic apparatus according to any one of claims 1 through 5, further comprising a display section for displaying tissue character values of the subject, wherein the display section simultaneously displays tissue character values determined from the motions of the tissue boundaries of the subject and the tissue character values computed in connection with the minute area of the fixed size in the subject.

7. The ultrasonic diagnostic apparatus according to claim 6, wherein the tissue character values determined from the motions of the tissue boundaries are numerals, and the tissue character values computed in connection with the minute area of the fixed size are displayed in the form of a distribution image.

8. The ultrasonic diagnostic apparatus according to any one of claims 1 through 7, further comprising:

a blood pressure value acquisition section that acquires a blood pressure value of the subject, wherein the tissue character value computing section computes at least either an elastic modulus or a viscosity as a tissue characteristic value of the subject from the motions of the tissue boundaries of the subject and the blood pressure value acquired by the blood pressure value acquisition section.

*FIG. 1*

EP 1 927 317 A1

# FIG. 2

TOMOGRAPHIC IMAGE 200

HIGH

HARD

VASCULAR WALL (ANTERIOR WALL ADVENTITIA)
VASCULAR WALL (ANTERIOR WALL MEDIA)
VASCULAR WALL (ANTERIOR WALL INTIMA)

Ws

VASCULAR LUMEN

VASCULAR WALL (POSTERIOR WALL INTIMA)
VASCULAR WALL (POSTERIOR WALL MEDIA)
VASCULAR WALL (POSTERIOR WALL ADVENTITIA)

LOW

SOFT

BLOOD FLOW-INTIMA
BOUNDARY 205
MEDIA-ADVENTITIA BOUNDARY 206

ROI204

POINT OF
MEASUREMENT
207

ELASTICITY SCALE 203
FOR ELASTICITY IMAGE

REFLECTION
INTENSITY
SCALE 202 FOR
TOMOGRAPHIC
IMAGE

EP 1 927 317 A1

FIG. 3

TOMOGRAPHIC IMAGE 200

HIGH — HARD

VASCULAR WALL (ANTERIOR WALL ADVENTITIA)
VASCULAR WALL (ANTERIOR WALL MEDIA)
VASCULAR WALL (ANTERIOR WALL INTIMA)

VASCULAR LUMEN

VASCULAR WALL (POSTERIOR WALL INTIMA)
VASCULAR WALL (POSTERIOR WALL MEDIA)
VASCULAR WALL (POSTERIOR WALL ADVENTITIA)

LOW — SOFT

REFLECTION INTENSITY SCALE 202 FOR TOMOGRAPHIC IMAGE

ROI204

ELASTICITY IMAGE 201

ELASTICITY SCALE 203 FOR ELASTICITY IMAGE

EP 1 927 317 A1

## FIG. 4

EP 1 927 317 A1

## FIG. 5

TOMOGRAPHIC IMAGE 200

HIGH

HARD

— VASCULAR WALL (ANTERIOR WALL ADVENTITIA)
— VASCULAR WALL (ANTERIOR WALL MEDIA)
— VASCULAR WALL (ANTERIOR WALL INTIMA)
— VASCULAR LUMEN

Ws

— VASCULAR WALL (POSTERIOR WALL INTIMA)
— VASCULAR WALL (POSTERIOR WALL MEDIA)
SOFT — VASCULAR WALL (POSTERIOR WALL ADVENTITIA)

LOW

BLOOD FLOW-INTIMA
BOUNDARY 205

MEDIA-ADVENTITIA BOUNDARY 206

ROI204

POINT OF
MEASUREMENT
207

ELASTICITY SCALE 203
FOR ELASTICITY IMAGE

REFLECTION
INTENSITY
SCALE 202 FOR
TOMOGRAPHIC
IMAGE

EP 1 927 317 A1

# FIG. 6

# FIG. 7

PROBE 101

SUBJECT

Ws

A
B
C

BLOOD VESSEL    ATHEROMA

ELECTROCARDIOGRAPHIC
COMPLEX

TRACED WAVEFORM
TA OF TISSUE A

TRACED WAVEFORM
TB OF TISSUE B

TRACED WAVEFORM
TC OF TISSUE C

THICKNESS CHANGE
WAVEFORM
WAB = TB−TA

THICKNESS CHANGE
WAVEFORM
WBC = TC−TB

R WAVEFORM

t [sec]

△ WAB

△ WBC

EP 1 927 317 A1

## FIG. 8

TOMOGRAPHIC IMAGE 200

HIGH — HARD

LOW — SOFT

REFLECTION INTENSITY SCALE 202 FOR TOMOGRAPHIC IMAGE

ELASTICITY IMAGE 201

ROI204

ELASTICITY SCALE 203 FOR ELASTICITY IMAGE

VASCULAR WALL (ANTERIOR WALL ADVENTITIA)
VASCULAR WALL (ANTERIOR WALL MEDIA)
VASCULAR WALL (ANTERIOR WALL INTIMA)
VASCULAR LUMEN
VASCULAR WALL (POSTERIOR WALL INTIMA)
VASCULAR WALL (POSTERIOR WALL MEDIA)
VASCULAR WALL (POSTERIOR WALL ADVENTITIA)

EP 1 927 317 A1

**INTERNATIONAL SEARCH REPORT**

| International application No. |
| --- |
| PCT/JP2006/318293 |

A. CLASSIFICATION OF SUBJECT MATTER
*A61B8/08*(2006.01)i

According to International Patent Classification (IPC) or to both national classification and IPC

B. FIELDS SEARCHED

Minimum documentation searched (classification system followed by classification symbols)
A61B8/08

Documentation searched other than minimum documentation to the extent that such documents are included in the fields searched
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| X<br>A | JP 2000-229078 A (Japan Science and Technology Corp.), 22 August, 2000 (22.08.00), Par. Nos. [0032] to [0035]; Fig. 1 (Family: none) | 1,2,8<br>5-7 |
| X<br>A | JP 2005-211590 A (Matsushita Electric Industrial Co., Ltd.), 11 August, 2005 (11.08.05), Par. Nos. [0037], [0043], [0044], [0066]; Figs. 1, 3 (Family: none) | 1-4,8<br>5-7 |
| A | JP 2000-271117 A (Aloka Co., Ltd.), 03 October, 2000 (03.10.00), Par. No. [0018]; Fig. 1 (Family: none) | 1,2 |

☒ Further documents are listed in the continuation of Box C.  ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 04 October, 2006 (04.10.06) | 24 October, 2006 (24.10.06) |

| Name and mailing address of the ISA/<br>Japanese Patent Office | Authorized officer |
| --- | --- |
| Facsimile No. | Telephone No. |

Form PCT/ISA/210 (second sheet) (April 2005)

**INTERNATIONAL SEARCH REPORT**

| International application No. |
| --- |
| PCT/JP2006/318293 |

C (Continuation). DOCUMENTS CONSIDERED TO BE RELEVANT

| Category* | Citation of document, with indication, where appropriate, of the relevant passages | Relevant to claim No. |
| --- | --- | --- |
| A | JP 2889568 B2 (Masao ITO, Yoshimitsu YAMAZAKI), 19 February, 1999 (19.02.99), Full text; Fig. 6 & US 6132373 A1 full text; Fig. 6 & EP 958784 A1 full text; Fig. 6 | 1,2 |
| A | JP 2004-215968 A (Matsushita Electric Industrial Co., Ltd.), 05 August, 2004 (05.08.04), Full text; Figs. 6, 7 (Family: none) | 5 |
| A | JP 2004-357892 A (Shimadzu Corp.), 24 December, 2004 (24.12.04), Full text; Fig. 4 (Family: none) | 6,7 |
| A | JP 2000-60853 A (Hitachi Medical Corp.), 29 February, 2000 (29.02.00), Full text; Fig. 3 (Family: none) | 6,7 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 10005226 A
- JP 2000229078 A
- JP 2005272571 A